(19) Europäisches Patentamt
European Patent Office
Office européen des brevets

(11) **EP 4 479 959 B1**

(12) **EUROPÄISCHE PATENTSCHRIFT**

(45) Veröffentlichungstag und Bekanntmachung des Hinweises auf die Patenterteilung:
**14.01.2026 Patentblatt 2026/03**

(21) Anmeldenummer: **23704085.2**

(22) Anmeldetag: **07.02.2023**

(51) Internationale Patentklassifikation (IPC):
**G08B 21/06** (2006.01)   **A61B 5/245** (2021.01)
**G01R 33/20** (2006.01)

(52) Gemeinsame Patentklassifikation (CPC):
**G08B 21/06; A61B 5/18; A61B 5/245; B60K 28/06; B60K 28/066; B60W 30/143; B60W 30/16; B60W 40/08; B60W 50/12; B60W 60/007; G01R 33/26;** A61B 5/4094; A61B 5/746; A61B 2562/0219

(86) Internationale Anmeldenummer:
**PCT/EP2023/052890**

(87) Internationale Veröffentlichungsnummer:
**WO 2023/156245 (24.08.2023 Gazette 2023/34)**

(54) **VERFAHREN ZUR ÜBERWACHUNG EINES FAHRERZUSTANDS**

METHOD FOR MONITORING THE STATE OF A DRIVER

PROCÉDÉ DE SURVEILLANCE DE L'ÉTAT D'UN CONDUCTEUR

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **18.02.2022 DE 102022201707**

(43) Veröffentlichungstag der Anmeldung:
**25.12.2024 Patentblatt 2024/52**

(73) Patentinhaber: **Robert Bosch GmbH**
**70442 Stuttgart (DE)**

(72) Erfinder:
• **STUERNER, Felix Michael**
**89077 Ulm (DE)**
• **FUCHS, Tino**
**72076 Tuebingen (DE)**
• **CIPOLLETTI, Riccardo**
**73312 Geislingen An Der Steige (DE)**
• **KRETSCHMANN, Andre Rudi**
**72116 Moessingen (DE)**
• **BIEG, Hans-Joachim**
**71093 Weil Im Schoenbuch (DE)**
• **WANIEK, Nicolai**
**89160 Dornstadt (DE)**

(56) Entgegenhaltungen:
**US-A1- 2015 219 732   US-A1- 2017 305 349
US-A1- 2020 057 115**

**Beschreibung**

Die vorliegende Erfindung betrifft ein Verfahren zur Zustandsüberwachung eines Insassen in einem Fahrzeug sowie eine Recheneinheit und ein Computerprogramm zu dessen Durchführung.

Hintergrund der Erfindung

Es sind verschiedene Verfahren und Systeme bekannt, um Fahrerzustände zu überwachen. So können beispielsweise Augenbewegungen und Blinzelfrequenzen durch Kamerabilder überwacht werden und aus bestimmten Mustern dann auf die Fahrtüchtigkeit des Nutzers geschlossen werden. Dazu muss aber durchgehend ein freies Sichtfeld vorhanden sein, was beispielsweise durch schlechte Lichtverhältnisse, Kopfbewegungen oder Brillen verhindert werden kann.

Darüber hinaus bieten Messmethoden wie das EEG (Elektroenzephalogramm), bei dem Potentialänderungen über die Kopfhaut verteilt gemessen werden, zuverlässige Hinweise auf den mentalen Zustand eines Nutzers, unter anderem zur Erkennung von Müdigkeit oder Notfällen. Jedoch müssen dabei Elektroden am Kopf korrekt angelegt und für ein gutes Messergebnis mit Kontaktgel versehen werden, so dass ein EEG als Messverfahren im Alltag wie etwa zur Fahrerüberwachung nicht praktikabel ist.

Außerdem sind im Automobilbereich mittlerweile Systeme wie eCall weit verbreitet, die bei Unfällen eines Fahrzeugs eine automatische Alarmierung von Rettungskräften über das Mobilfunknetz auslösen können und dabei Positionsdaten und Fahrzeugdaten übermitteln können. Allerdings kommen diese Systeme erst dann zur Anwendung, wenn ein Unfall bereits eingetreten ist und anhand eines Aufpralls oder anderer Fahrzeugparameter erkannt wurde.

Die US 2017/305 349 offenbart u.a. ein Verfahren zur Zustandsüberwachung eines Fahrzeuginsassen. Hierbei werden im Kopfbereich des Insassen Sensordaten durch eine oder mehrere Magnetenzephalographie-Sensoreinheiten erfasst. Es wird anhand der Sensordaten geprüft, ob Hinweise auf einen unerwünschten oder unbekannten Zustand des Insassen, der die Fahrtüchtigkeit einschränkt, vorliegen. Ist dies der Fall, wird ein zustandsabhängiges Signal in dem Fahrzeug ausgegeben. Die US 2020/0057115 offenbart eine Messvorrichtung, mit der im Kopfbereich einer Person Sensordaten erfasst werden können, die Feldstärken von magnetischen Feldern an jeweils räumlich unterschiedlichen Stellen im Kopfbereich darstellen. Mit Hilfe eines Gradiometers wird dann mindestens ein Gradient aus den erfassten Feldstärken gebildet, so dass ein magnetisches Hintergrundfeld im Wesentlichen eliminiert wird.

Die US 2015/0219732 offenbart ebenfalls ein Messsystem zur Erfassung von Sensordaten im Kopfbereich einer Person.

Offenbarung der Erfindung

Erfindungsgemäß werden ein Verfahren zur Zustandsüberwachung eines Insassen, insbesondere Fahrers, in einem Fahrzeug sowie eine Recheneinheit und ein Computerprogramm zu dessen Durchführung mit den Merkmalen der unabhängigen Patentansprüche vorgeschlagen. Vorteilhafte Ausgestaltungen sind Gegenstand der Unteransprüche sowie der nachfolgenden Beschreibung.

Insbesondere wird ein Verfahren vorgeschlagen, bei dem zunächst über eine oder mehrere Sensoreinheiten im Kopfbereich eines Insassen Sensordaten erfasst werden, die abhängig von einem mentalen oder gesundheitlichen Zustand des Insassen sind. Das Erfassen von Sensordaten erfolgt über eine oder mehrere Sensoreinheiten im Kopfbereich des Insassen, wobei die Sensordaten mindestens zwei Feldstärken von magnetischen Feldern an jeweils räumlich unterschiedlichen Stellen im Kopfbereich des Insassen umfassen. Es wird mindestens ein Gradient aus den mindestens zwei erfassten Feldstärken gebildet, so dass ein magnetisches Hintergrundfeld im Wesentlichen eliminiert wird. Dann wird geprüft, ob der gebildete Gradient auf einen unerwünschten oder unbekannten Zustand des Insassen hinweist, der die Fahrtüchtigkeit des Insassen einschränkt, und falls dies der Fall ist, wird eine zustandsabhängige Reaktion in dem Fahrzeug ausgelöst.

Bevorzugt umfassen die ein oder mehreren Sensoreinheiten mindestens einen spinresonanzbasierten Magnetfeldsensor zum Erfassen von durch Hirnströmen induzierten Hirn-Magnetfeldern und/oder ein Gyroskop zum Erfassen von Kopfbewegungen. Durch geeignete empfindliche Magnetfeldsensoren, insbesondere durch spinresonanzbasierte Sensoren wie NV-Zentren-Magnetometer oder Dampfzellen-Magnetometer, können indirekt über die induzierten Felder zeitliche Verläufe von Hirnstrommustern erfasst werden, die dann durch geeignete Mustererkennung mit bestimmten Zuständen verknüpft werden können. Dazu können die Magnetometer direkt am Kopf angebracht werden oder nur in der Nähe des Kopfes, etwa in einer Kopfstütze. Auf ähnliche Weise können auch Gyroskopdaten eines Gyroskops, insbesondere Dampfzellen-Gyroskops, das am Kopf des Insassen angebracht werden kann, genutzt werden, um anhand spezifischer Bewegungsmuster bestimmte Zustände zu erkennen. Mögliche unerwünschte Zustände, die mit einem solchen System erkannt werden können, sind beispielsweise Müdigkeit, Einschlafen, erhöhtes Stresslevel, niedrige Konzentration, aber auch eine neuronale Erkrankung, ein epileptischer Anfall oder ein Schlaganfall.

Damit ergibt sich eine einfache Möglichkeit, kritische Fahrerzustände sehr frühzeitig zu erkennen und geeignet einzugreifen, um Unfälle zu verhindern oder im Notfall schnelle Hilfe von außen anzufordern. Im Gegensatz zu Systemen wie einer Aufprallerkennung kann hier bereits reagiert werden, bevor eine Unfallsituation eintritt. Die insbesondere alltagstaugliche Möglichkeit zur Auswertung von hirnstromabhängigen Signalen erlaubt das Erkennen und sinnvolle Unterscheiden von spezifischen Fahrerzuständen, wie etwa Erkrankungen oder Sekundenschlaf. Entsprechend können auch die Reaktionen an diese verschiedenen Zustände angepasst werden, während eine einfache Blinzelerkennung oder sonstige bekannte Überwachungsmethoden keine weitere Einordnung des Problems ermöglichen.

Um in einer Alltagsumgebung wie einem Fahrzeug einsetzbar zu sein, sollen Magnetfelder, die nicht von der Hirnaktivität stammen, aus der Messung möglichst eliminiert werden. Gerade im Automobilbereich entstehen im Hintergrund vergleichsweise hohe und kaum abgeschirmte Magnetfelder, die etwa im Bereich von $10^{-6}$ bis $10^{-9}$ Tesla (Nanotesla) liegen, zusätzlich ist das Erdmagnetfeld im Bereich von $10^{-5}$ Tesla (einige Mikrotesla) vorhanden. Dagegen betragen die biomagnetischen Felder, die hier von Interesse sind, im Bereich von $10^{-12}$ Tesla (Picotesla) oder kleiner.

Die Elimination der Hintergrundmagnetfelder kann beispielsweise durch eine Gradiometeranordnung bei der Magnetfeldmessung erreicht werden. Als Gradiometer werden grundsätzlich Sensoreinheiten bezeichnet, die in der Lage sind, nicht nur die Feldstärke, sondern auch den Gradienten des Felds zu erfassen.

Dazu können mindestens zwei einzelne Magnetometer verwendet werden, die an räumlich unterschiedlichen Stellen angeordnet sind. Aus den erfassten einzelnen Feldsignalen wird eine Differenz gebildet. Durch eine versetzte Anordnung der Magnetometer relativ zu einer Magnetfeldquelle, also in diesem Fall gegenüber verschiedenen Hirnbereichen eines Fahrers, können die an beiden Orten näherungsweise gleich starken Hintergrundfelder bei der Signaldifferenzbildung im Wesentlichen eliminiert werden, während sich die interessierenden schwachen Felder mit dem Quadrat des Abstands von der Quelle verringern. Der Gradient entspricht damit also näherungsweise dem interessierenden schwachen Magnetfeld. Zu diesem Zweck können beispielsweise zwei Magnetometer übereinander in einer axialen Gradiometerkonfiguration, d.h. im Wesentlichen senkrecht zur Schädeloberfläche, oder nebeneinander angeordnet werden.

Die zustandsabhängige Reaktion in dem Fahrzeug kann beispielsweise das Ausgeben eines optischen oder akustischen Signals umfassen. Damit könnte sowohl der Fahrer, dessen Zustand überwacht wird, auf seinen Zustand aufmerksam gemacht werden (z.B. Müdigkeit); ebenso können damit aber auch Hinweise an Mitfahrer ausgegeben werden.

Zusätzlich oder alternativ kann das optische oder akustische Signal einen Hinweis auf die Art der auszulösenden zustandsabhängigen Reaktion und/oder einen Hinweis auf den erkannten Zustand des Insassen umfassen. So kann der Fahrer beispielsweise vor einem plötzlichen Bremsmanöver oder der Übernahme bestimmter Fahrfunktionen gewarnt werden, oder andere Personen können direkt informiert werden, welche Art von kritischem Zustand erkannt wurde. Optional kann vorgesehen sein, dass die zustandsabhängige Reaktion nicht ausgelöst wird, falls ein Fahrerbefehl zum Abbrechen der Reaktion erfasst wird. Dies kann auch mit den beschriebenen Hinweisen über ein optisches oder akustisches Signal kombiniert werden, so dass der Fahrer eine Möglichkeit hat, die Reaktion zu verhindern bzw. abzubrechen.

Erfindungsgemäß umfaßt die zustandsabhängige Reaktion eines der folgenden oder eine Kombination davon, nämlich das Aktivieren einer autonomen oder teilautonomen Fahrfunktion des Fahrzeugs, das Deaktivieren einer autonomen oder teilautonomen Fahrfunktion des Fahrzeugs oder das Verändern von Betriebsparametern einer autonomen oder teilautonomen Fahrfunktion des Fahrzeugs. Damit kann unmittelbar in den Fahrbetrieb eingegriffen werden, um gefährliche Situationen abzuwenden. Geeignete autonome oder teilautonome Fahrfunktion des Fahrzeugs könnten beispielsweise eine Abstandsregelung zum Regeln eines Abstands des Fahrzeugs von anderen Objekten, eine Regelung der Fahrgeschwindigkeit des Fahrzeugs oder eine vollautonome Steuerung des Fahrzeugs umfassen. Damit kann ein Fahrzeug, das mit solchen Funktionen ausgestattet ist, ein automatisches Bremsmanöver, ein sicheres Steuern in eine Parksituation oder ein Verringern der Geschwindigkeit auslösen.

Zusätzlich kann die zustandsabhängige Reaktion auch ein Übermitteln einer Nachricht über eine Kommunikationsschnittstelle umfassen.

Als Nachricht kommen dabei alle Formen der Datenübermittlung in Frage, deren genaue Ausgestaltung auch von den verwendeten Protokollen und Systemen abhängt. Ebenso können beliebige lang- oder kurzreichweitige Kommunikationstechnologien verwendet werden.

Insbesondere kann eine solche Nachricht zumindest einen Teil der erfassten Sensordaten und/oder Informationen über die erfassten Sensordaten umfassen. Dies ermöglicht es, die Daten an einem anderen Ort manuell oder automatisch weiter auszuwerten, um beispielsweise genauere Hinweise auf den gesundheitlichen Zustand des Insassen zu finden.

Eine Möglichkeit besteht darin, eine Nachricht an weitere Fahrzeuge zu übermitteln, die sich in der Umgebung des Fahrzeugs befinden. Dazu können auch bereits vorhandene Systeme wie eine Vehicle-to-Vehicle-Schnittstelle genutzt werden. Auch eine Alarmierung von Helfern in der Nähe wird damit möglich. Generell kann die Nachricht auch Informationen über das Aktivieren, Deaktivieren oder Verändern einer Fahrfunktion umfassen, um beispielsweise nachfolgende Fahrzeuge über zu erwartende Fahrmanöver zu informieren oder um diesen Fahrzeugen Daten bereitzustellen, die eine ebenfalls automatische Anpassung von Fahrfunktionen dieser übrigen Fahrzeuge ermöglichen.

Ebenso kann die Nachricht eine Notrufnachricht an eine Notrufzentrale umfassen, die über ein eigenes System oder auch über vorhandene Systeme wie eCall übermittelt wird.

Optional könnten mehrere Gruppen von möglichen Zuständen eines Insassen vorgegeben sein, wobei jeder Gruppe eine oder mehrere Reaktionen zugeordnet sein können.

Eine erfindungsgemäße Recheneinheit, z.B. ein Steuergerät eines Kraftfahrzeugs, ist, insbesondere programmtechnisch, dazu eingerichtet, ein erfindungsgemäßes Verfahren durchzuführen.

Auch die Implementierung eines erfindungsgemäßen Verfahrens in Form eines Computerprogramms oder Computerprogrammprodukts mit Programmcode zur Durchführung aller Verfahrensschritte ist vorteilhaft, da dies besonders geringe Kosten verursacht, insbesondere wenn ein ausführendes Steuergerät noch für weitere Aufgaben genutzt wird und daher ohnehin vorhanden ist. Schließlich ist ein maschinenlesbares Speichermedium vorgesehen mit einem darauf gespeicherten Computerprogramm wie oben beschrieben. Geeignete Speichermedien bzw. Datenträger zur Bereitstellung des Computerprogramms sind insbesondere magnetische, optische und elektrische Speicher, wie z.B. Festplatten, Flash-Speicher, EEPROMs, DVDs u.a.m. Auch ein Download eines Programms über Computernetze (Internet, Intranet usw.) ist möglich. Ein solcher Download kann dabei drahtgebunden bzw. kabelgebunden oder drahtlos (z.B. über ein WLAN-Netz, eine 3G-, 4G-, 5G- oder 6G-Verbindung, etc.) erfolgen.

Weitere Vorteile und Ausgestaltungen der Erfindung ergeben sich aus der Beschreibung und der beiliegenden Zeichnung.

Die Erfindung ist anhand von Ausführungsbeispielen in der Zeichnung schematisch dargestellt und wird im Folgenden unter Bezugnahme auf die Zeichnung beschrieben.

Kurze Beschreibung der Zeichnungen

Figur 1 zeigt schematisch den Aufbau einer möglichen Sensoreinheit, die in einem erfindungsgemäßen Verfahren verwendet werden kann;

Figur 2 zeigt beispielhaft eine Kopfstütze, in der Sensoreinheiten zum Erfassen von Daten im Kopfbereich des Insassen vorgesehen sind;

Figur 3 zeigt eine Abfolge möglicher Verfahrensschritte gemäß einer Ausführungsform; und

Figur 4 zeigt ein System, in dem die hier vorgestellten Verfahren ausgeführt werden können.

Ausführungsformen der Erfindung

Der mentale oder gesundheitliche Zustand eines Fahrers in einem Fahrzeug kann über nicht-invasive Sensoren überwacht werden, die am Kopf des Fahrers oder in der Nähe des Kopfes angebracht werden. Abhängig von den erfassten Zustandsdaten können verschiedene Reaktionen eingeleitet werden, um die Sicherheit des Fahrers und anderer Verkehrsteilnehmer zu gewährleisten.

Insbesondere können sich aus den Hirnströmen eines menschlichen Insassen, die beispielsweise über Kopfelektroden gemessen werden können, Hinweise auf verschiedene mentale, körperliche oder gesundheitliche Zustände des Insassen ergeben. Die elektrische Aktivität in den Hirnneuronen induziert aber auch ein schwaches Magnetfeld, das durch hochempfindliche Magnetfeldsensoren erfasst und ausgewertet werden kann. Aus dem zeitlichen Verlauf der induzierten Magnetfelder können dann ebenfalls Rückschlüsse auf die Hirnstrommuster und damit auf verschiedene Zustände eines Insassen gezogen werden. Die erfassten Magnetfelder können zu diesem Zweck auf der Grundlage bekannter Muster oder Musterparameter oder mit Hilfe trainierter Klassifikatoren ausgewertet werden.

Solche Sensoreinheiten oder Teile davon können insbesondere in einem Fahrzeug angebracht werden, z.B. in einer Kopfstütze, oder in Kopfbedeckungen oder anderen Halteelementen, die ein Fahrer am oder auf dem Kopf tragen kann. Beispielsweise können Sensoreinheiten auch in Brillenbügeln, Kopfbedeckungen wie Helmen oder Stirnbändern, oder an Ohrbügeln getragen werden.

Da die zu messenden Magnetfeldstärken für eine Überwachung der biomagnetischen Hirn-Magnetfelder sehr klein sind, eignen sich als Sensoren für einen derartigen Aufbau insbesondere quantenbasierte bzw. optisch gepumpte Magnetometer, die hochempfindliche Messungen erlauben. Dabei werden in den folgenden Ausführungsformen nur beispielhaft zwei Sensortypen als mögliche Magnetometer für die vorgeschlagene Sensoreinheit beschrieben, nämlich Dampfzellen-Magnetometer und Diamant-NV-Magnetometer. Es ist jedoch auch denkbar, andere Magnetometer mit vergleichbaren Eigenschaften in die Sensoreinheit zu integrieren.

Die hier beispielhaft beschriebenen Magnetometer nutzen optisch gepumpte und/oder optisch detektierte magnetische Resonanzen (optically detected magnetic resonance, ODRM). Dabei wird ausgenutzt, dass unter Einfluss eines äußeren Magnetfelds die Energieniveaus bestimmter Spinzustände ungepaarter Elektronen aufspalten (Zeeman-Effekt). Durch die Aufspaltung der Energieniveaus ergeben sich veränderte Übergänge bei der Relaxation aus angeregten Zuständen, die dann beispielsweise durch optische Anregung und frequenzabhängige Detektion der resultierenden Fluoreszenzstrahlung oder durch Beobachtung optischer Eigenschaften wie der Absorption von Licht gemessen werden können. Aus den gemessenen optischen Parametern kann dann wiederum auf die Magnetfeldstärke geschlossen werden.

Diamant-NV-Magnetometer beruhen auf dem optischen Auslesen von Spin-Magnetresonanzen an speziellen Defektzentren in Diamant, insbesondere von Stickstoff-Fehlstellen (NV, nitrogen vacancy), die als Verunreinigungen des Kohlenstoffgitters von Diamant auftreten und auch gezielt eingebracht werden können. Details zu einer möglichen Ausführungsform eines Diamant-NV-Magnetometers finden sich beispielsweise in der DE 10 2018 202 238 A1.

Dampfzellen-Magnetometer beruhen ebenfalls auf der Auslesung von Spinresonanzen. Dabei wird eine Zelle, z.B. eine geschlossene Kavität in einem Silizium-Wafer, mit gasförmigen Atomen und gegebenenfalls zusätzlichen Puffergasen gefüllt, z.B. verdampfte Alkalimetalle wie Kalium, Rubidium oder Cäsium, die ebenfalls ungepaarte Elektronen aufweisen. Je nach Ausführungsform können auch Mischungen aus Alkali-Dampf und Edelgasen verwendet werden. In anderen Fällen werden reine Edelgase genutzt.

Auch bei Dampfzellen-Magnetometern wird eine Spinpolarisierung über optisches Pumpen mit einer Anregungslichtquelle erreicht. Anschließend werden optische Eigenschaften der Atome in der Zelle über die Resonanzeffekte ausgelesen, z.B. mit einer zusätzlichen Auslese-Lichtquelle, anhand derer die Absorption des Ausleselichts durch die Dampfzelle gemessen wird. Dabei können unterschiedliche magnetfeldabhängige Zustandsaufspaltungen beobachtet werden.

Um in einer Alltagsumgebung wie einem Fahrzeug einsetzbar zu sein, sollen Magnetfelder, die nicht von der Hirnaktivität stammen, aus der Messung möglichst eliminiert werden. Gerade im Automobilbereich entstehen im Hintergrund vergleichsweise hohe und kaum abgeschirmte Magnetfelder, die etwa im Bereich von $10^{-6}$ bis $10^{-9}$ Tesla (Nanotesla) liegen, zusätzlich ist das Erdmagnetfeld im Bereich von $10^{-5}$ Tesla (einige Mikrotesla) vorhanden. Dagegen bewegen sich die biomagnetischen Felder, die hier von Interesse sind, im Bereich von $10^{-12}$ Tesla (Picotesla) oder noch darunter.

Die Elimination der Hintergrundmagnetfelder kann beispielsweise durch eine Gradiometeranordnung bei der Magnetfeldmessung erreicht werden. Als Gradiometer werden grundsätzlich Sensoreinheiten bezeichnet, die in der Lage sind, nicht nur die Feldstärke, sondern auch den Gradienten des Felds zu erfassen.

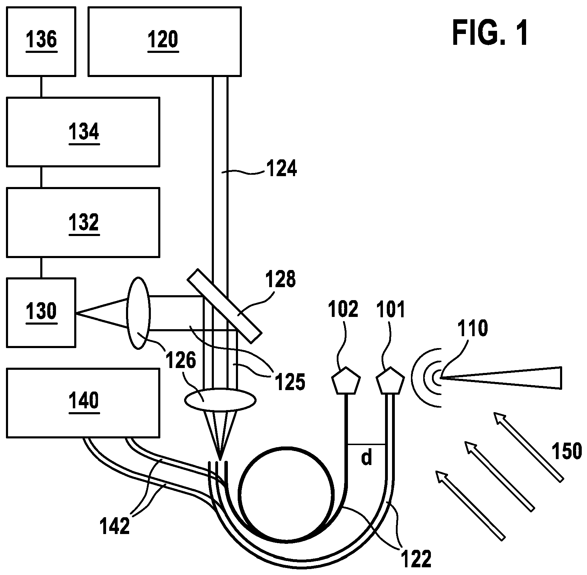

Ein Beispiel eines Gradiometers auf Basis von NV-Zentren-Magnetometern ist in Figur 1 gezeigt. Jedoch gelten die nachfolgenden Gradiometereigenschaften im Wesentlichen auch für Gradiometer anderer Bauart oder mit anderen Magnetomter-Typen.

Dazu können mindestens zwei einzelne Magnetometer verwendet werden, die an räumlich unterschiedlichen Stellen angeordnet sind. Aus den erfassten einzelnen Feldsignalen wird eine Differenz gebildet. Durch eine versetzte Anordnung der beiden Sensorköpfe, z.B. der Sensor-Diamanten 101 und 102 eines NV-Zentren-Magnetometers relativ zur Magnetfeldquelle 110, also in diesem Fall gegenüber der verschiedenen Hirnbereiche eines Fahrers, können die an beiden Orten näherungsweise gleich starken Hintergrundfelder bei der Signaldifferenzbildung im Wesentlichen eliminiert werden, während sich die interessierenden schwachen Felder mit dem Quadrat des Abstands von der Quelle verringern. Der Gradient entspricht damit also näherungsweise dem interessierenden schwachen Magnetfeld. Zu diesem Zweck können beispielsweise zwei Magnetometer übereinander in einer axialen Gradiometerkonfiguration, d.h. im Wesentlichen senkrecht zur Schädeloberfläche, angeordnet werden, wobei nur als Beispiel zwischen den beiden Sensorköpfen (z.B. den Sensordiamanten eines NV-Zentren-Magnetometers) ein Abstand d von einigen mm bis cm liegen kann. In anderen Ausführungen können auch zwei Sensorköpfe 101, 102 nebeneinander angeordnet werden. Der Abstand der Sensoreinheit vom Kopf kann ebenfalls im mm- bis cm-Bereich liegen.

Durch Verwendung derselben Lichtquelle 120 für das Anregungslicht 124 und derselben Mikrowellenquelle 140 zur Resonanzerzeugung bei einem NV-Zentren-Magnetometer kann erreicht werden, dass Rauschanteile aus diesen Quellen durch die Differenzsignalbildung automatisch eliminiert werden, da dieses Rauschen bzw. Schwankungen an beiden Sensorköpfen gleich auftritt. Das Anregungslicht 124 kann ebenso wie das Fluoreszenzlicht 125 über Faseroptiken 122 und verschiedene optische Elemente wie Strahlteiler, Linsen oder Spiegel 126, 128 geleitet werden. Das Fluoreszenzlicht wird schließlich von einem Photodetektor 130 detektiert und das Signal in weiteren Signalverarbeitungsstufen 132 bis 136 nachverarbeitet, wie etwa einem Differenzverstärker, einem AD-Wandler, Frequenzfiltern und Mustererkennungseinheiten.

Zusätzlich oder alternativ zu einer Gradiometerkonfiguration kann für ein Magnetometer eine Lock-In-Detektion genutzt werden, um die unerwünschten Hintergrundfelder aus der Messung zu eliminieren.

Als weitere Ausführungsform können auch Gradiometereinheiten zur Fahrerzustandsüberwachung aus zwei Dampfzellen-Magnetometern gebildet werden. Während diese Dampfzellen-Magnetometer oft eine starke magnetische Abschirmung benötigen, da sie üblicherweise nur bei kleinen Feldstärken nahe dem feldfreien Bereich (near-zero field) betrieben werden, können mit einer Gradiometerkonfiguration oder mit geeigneter Lock-In-Detektion auch Messungen in Normalumgebung mittels Dampfzellen vorgenommen werden. Ebenso sind auch Kombinationen aus Dampfzellen und NV-Zentren-Magnetometern innerhalb einer Gradiometereinheit möglich.

Um durch Hirnströme induzierte Magnetfelder auszulesen, können dann mehrere einzelne Magnetometer und/oder mehrere Gradiometer-Einheiten aus jeweils zwei Magnetometern an verschiedenen Stellen rund um den Kopf eines Fahrers angeordnet werden, um zusammen eine Sensoreinheit zu bilden. Entsprechend ihrer spezifischen räumlichen Anordnung addieren sich die von einzelnen Neuronen erzeugten Potentiale und die induzierten Magnetfelder auf, so dass sich über den gesamten Kopf verteilte Magnetfeldänderungen messen lassen. Grundsätzlich kann dazu beispielsweise eine Art Kopfbedeckung oder eine am Kopf getragene Haltevorrichtung für die ganze Sensoreinheit oder für Teile der Sensoreinheit, also z.B. für die mehreren Magnetometer oder Gradiometereinheiten, verwendet werden. Falls ein Nutzer einen Helm trägt, wie als Motorradfahrer oder Pilot, könnten eine oder mehrere Sensoreinheiten, Gradiometereinheiten oder Magnetometer-Sensorköpfe direkt in diesen Helm integriert werden. Auch Brillenbügel, Ohrbügel, Hörgeräte und andere am Kopf getragenen Vorrichtungen können mit den hier beschriebenen Sensoreinheiten kombiniert werden und die Sensorköpfe an geeigneter Stelle aufnehmen. Außerdem können Haltevorrichtungen in der Form einer durchgehenden oder netzartigen Haube oder z.B. ähnlich einem Haarreif oder Stirnband verwendet werden, in denen mehrere Sensorköpfe untergebracht sind und die auf den Kopf aufgesetzt werden können.

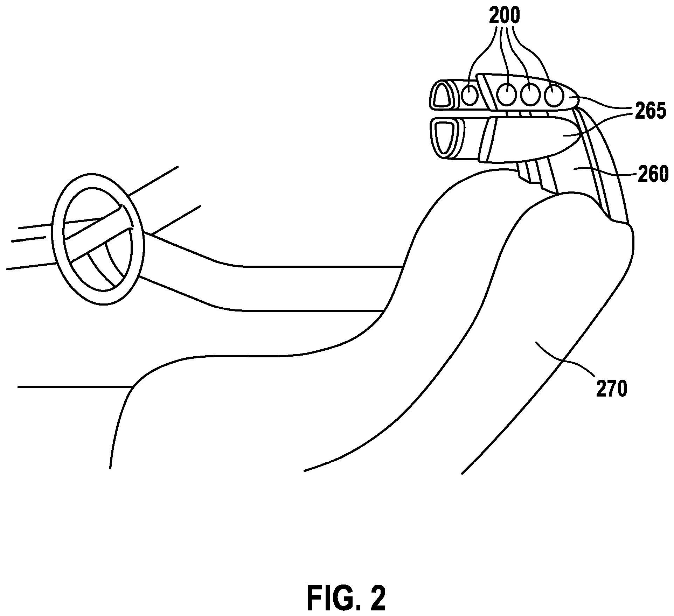

Alternativ können Magnetfeldsensoren in der Nähe des Kopfes angeordnet sein, ohne unmittelbar daran befestigt zu sein. Zur Überwachung des Zustands eines Insassen in einem Fahrzeug können Sensoreinheiten oder Teile davon beispielsweise in einer Kopfstütze 260 eines Sitzelements 270 integriert sein, wie beispielhaft in Figur 2 gezeigt ist. Dabei können Ausläufer 265 der Kopfstütze seitlich und/oder oberhalb des Kopfes zumindest teilweise um den Kopf herumreichen und in diesen Seitenteilen 265 mehrere Gradiometer 200 oder Magnetometer an verschiedenen Stellen angeordnet sein. Es versteht sich, dass dabei die Ausformungen der Kopfstütze bzw. der Seitenteile 265 der Kopfstütze bevorzugt etwa der Kopfform folgen, um die Sensoren 200 möglichst nah an die erwünschten Messbereiche bringen zu können. Falls es für die gewünschte Überwachung ausreichend ist, Magnetfeldsignale vor allem am Hinterkopf zu erfassen, kann auch eine standardmäßige Kopfstütze mit entsprechend eingelassenen Sensorköpfen genutzt werden. In allen Varianten können auch Magnetometer und/oder Gradiometer auf verschiedenen Höhen angeordnet sein. Ebenso könnten Teile der Kopfstütze beweglich, klappbar oder verschiebbar ausgestaltet sein, um ein komfortables Einnehmen der Sitzposition bei optimaler Lage der Sensoreinheiten am Kopf während der Fahrt zu ermöglichen.

Außerdem ist es auch möglich, mit einer oder mehreren am Kopf eines Insassen positionierten Gyroskopeinheiten Veränderungen der Kopfposition und/oder Kopfbewegungen zu erfassen und ebenfalls auf bestimmte Muster oder Auffälligkeiten hin auszuwerten. Auch dadurch können sich Hinweise auf einen bestimmten Zustand eines Fahrers ergeben. Ein oder mehrere Gyroskope können zu diesem Zweck unmittelbar am Kopf des Nutzers angeordnet werden, also wieder beispielsweise in Kopfbedeckungen, Stirnbändern, Helmen, Ohr- oder Brillenbügeln oder anderen Elementen. Diese können spezifisch als Halterungen für die Gyroskope vorgesehen sein oder können, wie bei einem Brillenbügel, auch anderen Zwecken zusätzlich dienen.

Dabei kommen grundsätzlich alle Typen von Gyroskopen in Betracht. Besonders geeignet sind beispielsweise die weit verbreiteten MEMS-Drehratensensoren (microelectromechanical system), die klein und ausreichend empfindlich sind. Diese Sensoren sind üblicherweise als komplettes Sensorsystem ähnlich einer integrierten Schaltung gebaut und können Sensoren für eine oder mehrere Achsen umfassen.

Ebenso können aber auch andere Gyroskope zur Zustandsüberwachung verwendet werden. Auf ähnliche Weise wie für die Anwendung als Magnetometer beschrieben, können Dampfzellen und NV-Zentren in Diamant auch zur spinbasierten optischen Messung von Drehraten genutzt werden und hochempfindliche Gyroskope bilden. Dabei wird in diesen Fällen die Spin-Larmorpräzession $\omega_{larmor}$ direkt ausgelesen. Eine äußere Rotation des Gyroskops stellt eine zusätzliche Drehung dar, welche durch Auslesen der Rotationsfrequenz $\omega_{mess}$ ermittelt werden kann,

$$\omega_{mess} = \omega_{larmor} \pm \omega_{rot}$$

Beispielsweise offenbart die DE 10 2019 219 061 A1 eine Möglichkeit zur Messung von drei Rotationsrichtungen mittels eines NMR-Gyroskops.

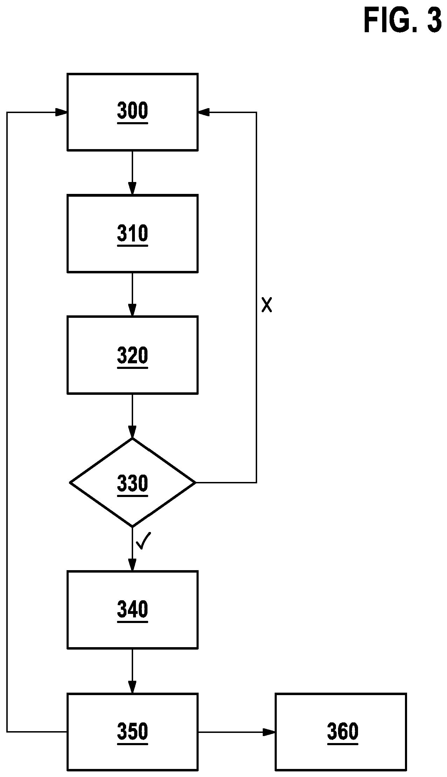

Figur 3 zeigt beispielhaft Verfahrensschritte gemäß einer möglichen Ausführungsform zur Zustandsüberwachung in einem Fahrzeug. Dabei werden in Schritt 300 zunächst Sensordaten der vorhandenen Sensoreinheiten erfasst. Optional kann sich daran eine Vorverarbeitung der erfassten Signale in Schritt 310 anschließen, wie etwa eine Verstärkung von Signalen, das Bilden von Differenzsignalen für ein Gradiometer, eine Signalfilterung, oder eine Korrektur der erfassten Signale auf Basis weiterer Daten, z.B. eine Korrektur von Magnetfelddaten abhängig von einer durch ein Gyroskop erfassten Kopfposition gegenüber den Magnetfeldsensoren. Anschließend kann in Schritt 320 der zeitliche Verlauf der Magnetfeldsignale (und/oder der Gyroskopsignale) in einer Mustererkennungseinheit ausgewertet werden, z.B. in Bezug auf bestimmte bekannte oder unbekannte Muster oder auf bestimmte Parametereigenschaften. Insbesondere wird dann in Schritt 330 geprüft, ob die Sensordaten auf unerwünschte Zustände des Insassen schließen lassen. Daran schließt sich eine Auswertungseinheit an, die aufgrund der erkannten Muster bestimmte Reaktionen in Schritt 340 auswählt und in Schritt 350 ansteuert. Dabei können sich die Mustererkennung, die Auswertung und Einordnung der erkannten Muster sowie die Auswahl der zugehörigen Reaktionen für ein erkanntes Muster auch überschneiden, so dass beide Funktionen von einer einzelnen Einheit übernommen werden oder anders aufgeteilt werden können. Es ist auch möglich, dass abhängig von der in Schritt 350 durchgeführten Reaktion eine weitere Reaktion 360, wie z.B. das Informieren des Nutzers oder anderer Fahrzeuge über die durchgeführte Reaktion, ausgelöst wird.

Zur Auswertung der erfassten Signale können klassische Methoden wie verschiedene Mustererkennungsverfahren, einfache Grenzwerte, zeitliche Verläufe, Toleranzbereiche, Steigungen, Frequenzauswertungen und andere zur Anwendung kommen. Die jeweiligen Referenzwerte können im System abgespeichert sein und bestimmten Zuständen oder Erkrankungen zugeordnet sein, wie etwa Müdigkeit, Stress, Schlaf, fehlender Aufmerksamkeit bzw. geringer Konzentration, aber auch neuronalen Erkrankungen wie Parkinson, Epilepsie oder z.B. Schlaganfällen.

Alternativ oder zusätzlich kann an dieser Stelle auch künstliches/maschinelles Lernen z.B. in Form eines neuronalen Netzes eingesetzt werden, um die gemessenen Signale auszuwerten und geeignet zu klassifizieren. Beispielsweise kann ein neuronales Netz ebenfalls mit im Labor gewonnenen Daten trainiert sein, die optional auch durch weitere Messmethoden wie EEG als Referenz bewertet wurden. Anhand dieser Daten kann dann eine künstlich lernende Einheit die aktuellen Sensordaten klassifizieren und darin wiederum beispielsweise Müdigkeit oder Stress erkennen.

Die durch die Sensoreinheiten, d.h. beispielsweise durch die Magnetometer oder durch die Gyroskope gewonnenen Daten können bevorzugt im laufenden Fahrbetrieb kontinuierlich- bzw. in vorgegebenen Abständen erfasst und ausgewertet werden. Falls dabei aufgrund der Magnetfeldmessungen und/oder auf Grundlage weiterer Überwachungsfunktionen ein verschlechterter oder kritischer Fahrerzustand erkannt wird, werden verschiedene Reaktionen des Systems eingeleitet.

Ebenso können auch Reaktionen ausgelöst werden, wenn der erfasste Fahrerzustand nicht eingeordnet werden kann, da beispielsweise das erfasste Magnetfeldmuster keinem bekannten oder erwarteten Muster - weder einem Normalzustand noch einem kritischen Zustand - entspricht.

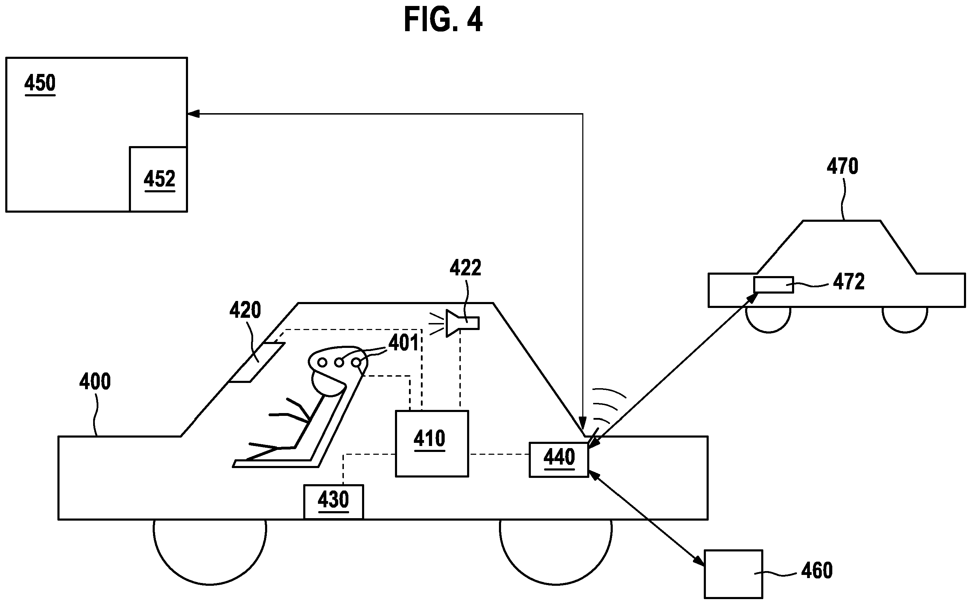

Figur 4 zeigt beispielhaft ein System, in dem Ausführungsformen des hier beschriebenen Verfahrens umgesetzt werden können. Dabei ist ein Fahrzeug 400 gezeigt, das mit Sensoreinheiten 401 zur Erfassung von zustandsspezifischen Sensordaten im Kopfbereich des Nutzers ausgestattet ist. Die erfassten Daten werden von einer Auswertungseinheit 410 verarbeitet, ausgewertet oder überprüft. Die weiteren Elemente des Systems werden nachfolgend anhand der Reaktionen beschrieben, die abhängig von einem Fahrerzustand ausgelöst werden.

Eine erste Gruppe von mögliche Reaktionen auf einen kritischen oder unbekannten Nutzerzustand umfasst solche Reaktionen, die unmittelbar im Fahrzeug ausgelöst werden. Eine einfache Möglichkeit besteht beispielsweise darin, bei erkannter Müdigkeit eines Fahrers ein akustisches oder optisches Warnsignal auszugeben, z.B. einen Warnton. Dieses kann dazu beitragen, dass der Fahrer aufmerksam bleibt. Alternativ oder zusätzlich könnten Verhaltenshinweise, die im System vorgespeichert sind, für den Fahrer als Textnachricht angezeigt oder hörbar ausgegeben werden, wie etwa eine Aufforderung, so bald wie möglich eine Fahrpause einzulegen. Insbesondere kann dabei die Einordnung des erkannten Zustands, wie etwa "Stress" oder "Müdigkeit", optional auch mit an den Fahrer ausgegeben werden.

Falls unmittelbare Gefahr droht, weil beispielsweise ein Zustand erkannt wird, in dem ein Nutzer nicht mehr bei Bewusstsein ist, können auch deutlichere Warnsignale im oder am Fahrzeug ausgelöst werden, wie etwa laute Alarmtöne oder Lichtsignale. Dabei können für alle ausgegebenen Signale bevorzugt Elemente genutzt werden, die im Fahrzeug bereits vorhanden sind, wie z.B. ein Kommunikations-und Navigationssystem mit entsprechenden Ausgabemöglichkeiten wie einem Display 420 und Lautsprechern 422 und geeigneter Software zur Sprachausgabe, oder eine Beleuchtungseinrichtung im oder am Fahrzeug. Es könnten aber auch eigene Elemente wie Lautsprecher oder Leuchten in dem Überwachungssystem integriert sein. Damit können sowohl Mitfahrende als auch Personen außerhalb des Fahrzeugs auf den Notfall aufmerksam gemacht werden. Auch hier können zusätzlich Text- oder Sprachausgaben genutzt werden, um die vermutete Art des Notfalls mitzuteilen oder Anweisungen zu geben.

Eine weitere mögliche Reaktion auf Grundlage der ausgewerteten Mess- und Zustandsdaten eines Fahrers besteht in der Ansteuerung verschiedener Fahrfunktionen, die dem Fahrer zur Verfügung stehen. Beispielsweise kann festgelegt werden, dass abhängig von einem erkannten Fahrerzustand wie Müdigkeit oder Stress die mögliche Höchstgeschwindigkeit des Fahrzeugs begrenzt oder gesenkt wird, um das Unfallrisiko zu senken. Eine solche Funktion kann als einfacher oberer Grenzwert vorgesehen sein, der auch bei anderer Anforderung durch den Fahrer nicht überschritten werden kann, oder als Teil einer Geschwindigkeitsregelanlage, die automatisch eine vorgegebene Geschwindigkeit einhält.

Da im Fahrzeug ein oder mehrere Fahrerassistenzsysteme vorhanden sind, also teil- oder vollautonome Fahrfunktionen wie etwa ein Tempomat, eine Abstandsregelung, ein Notbremssystem oder andere, werden diese abhängig von dem erkannten Fahrerzustand aktiviert, deaktiviert oder mit anderen Parametern eingestellt.

Beispielsweise kann vorgesehen sein, dass unterstützende Systeme wie Tempomat oder Abstandsregelung immer aktiviert werden, sobald ein Fahrerzustand festgestellt wird, der auf Müdigkeit hinweist. Die Fahrerassistenzsysteme oder allgemein alle Fahrfunktionen, die in eine zustandsabhängige Reaktion mit einbezogen werden können, können durch ein oder mehrere geeignete Steuereinheiten 430 angesteuert werden, wie etwa ein zentrales Steuergerät eines Fahrzeugs oder dedizierte Steuerungen für einzelne Funktionen mit entsprechenden Hardware- und Softwaremodulen.

Es ist auch möglich, dass Fahrfunktionen vollautonom übernommen werden, insbesondere wenn der erfasste Zustand des Fahrers auf einen Notfall hindeutet, in dem der Fahrer vermutlich nicht mehr zum Führen des Fahrzeugs fähig ist. Beispielsweise kann automatisch auf eine geringere Geschwindigkeit oder bis zum vollständigen Stillstand abgebremst werden, wenn Bewusstlosigkeit, ein epileptischer Anfall oder ähnliches erkannt wird. Falls das Fahrzeug Funktionen zum autonomen Fahren bietet, können diese alternativ oder zusätzlich als Reaktion auf einen erkannten Fahrerzustand aktiviert werden. Damit könnte beispielsweise eingeleitet werden, dass das Fahrzeug im Notfall selbständig in eine sichere Position fährt, etwa auf einen Standstreifen oder einen Parkplatz, oder zumindest stark befahrene Straßen verlässt.

Alternativ könnte auch vorgesehen sein, dass bestimmte Fahrfunktionen nur einem wachen Fahrer zugänglich sind. Beispielsweise sind viele autonome Fahrfunktionen nur unter der Voraussetzung zulässig, dass ein Nutzer die automatische Fahrt weiterhin überwacht und jederzeit eingreifen kann. In diesem Fall könnte festgelegt werden, dass ein als müde erkannter Fahrer keinen vollautonomen Fahrbetrieb nutzen darf und diese Funktion nicht mehr zur Verfügung steht, da die Gefahr des Einschlafens damit noch weiter erhöht wäre.

Die Einrichtungen, Sensoren und Steuerungen, die für solche teilautomatischen oder automatischen Fahrfunktionen und Assistenzsysteme 430 genutzt werden können, sind bekannt und werden hier nicht weiter ausgeführt. Es versteht sich, dass hier verschiedene Varianten genutzt werden können, z.B. Kameras, Ultraschallsensoren oder Lidar zur Erfassung von Umgebungsdaten, die dann auf geeignete Weise verarbeitet werden und in entsprechende Ansteuerung von Lenkung, Geschwindigkeitsregelung und anderen Elementen umgesetzt werden.

Es gibt außerdem verschiedene Möglichkeiten für fahrzeuggebundene Steuereinheiten, mit verschiedenen Stellen zu kommunizieren, also Daten und Nachrichten zu senden oder zu empfangen. Damit können beispielsweise automatisch Notrufsignale aus einem Fahrzeug übermittelt werden. Ein Fahrzeug bzw. eine geeignete Steuer- oder Recheneinheit im Fahrzeug kann mit eigenen Kommunikationsschnittstellen 440 ausgestattet sein, wie etwa einem Mobilfunkmodul, das eine Verbindung der Steuersoftware über ein Mobilfunknetz ermöglicht. Zusätzlich oder alternativ kann ein Fahrzeug auch lokale drahtlose oder drahtgebundene Schnittstellen (z.B. WLAN, Bluetooth) aufweisen, über die weitere Kommunikationsgeräte wie z.B. ein Mobiltelefon angebunden werden können, so dass eine indirekte Kommunikationsverbindung über diese separaten Geräte möglich wird.

Darüber hinaus sind auch Systeme verfügbar, die über kurz- oder mittelreichweitige Funkschnittstellen oder z.B. ebenfalls über Mobilfunkschnittstellen eine Kommunikation zwischen Fahrzeugen ermöglichen, die sich in derselben Umgebung befinden (Vehicle-to-Vehicle, V2V). Auf ähnliche Weise können Fahrzeuge mit Infrastruktureinrichtungen kommunizierend verbunden sein (Vehicle-to-Infrastructure, V2I).

Falls ein kritischer oder ungünstiger Zustand eines Fahrers bei der Auswertung der Magnetfeld- und/oder Gyroskopsignale erkannt wird, können solche Kommunikationsschnittstellen 440 auf verschiedene Weise eingesetzt werden.

Bei einem erkannten medizinischen Notfall kann beispielsweise automatisch ein Notruf an eine Rettungsleitstelle oder eine andere Zentraleinheit 450 übermittelt werden, wobei in der Notrufnachricht Daten aus anderen Einrichtungen im Fahrzeug wie die momentane Fahrzeugposition angegeben werden können. Es könnten auch weitere Daten für solche Fälle vorgespeichert sein, z.B. Gesundheitsdaten eines Fahrers mit bekannten Vorerkrankungen oder Notfallkontakte, die dann ebenfalls bei Bedarf mit übermittelt werden können.

Da das erfindungsgemäße System in der Lage sein kann, einen sich abzeichnenden Notfall schon frühzeitig zu erkennen, kann alternativ oder zusätzlich auch eine automatische Sprechverbindung über geeignete Kommunikationskanäle zu einer Rettungsleitstelle 450 aufgebaut werden, so dass eine Person den Fahrer aktiv zu seinem Zustand befragen kann. Damit kann, falls der Fahrer noch ansprechbar ist, das Problem genauer eingegrenzt werden. Außerdem können auf diese Weise Fehlalarme des Systems auf einfache Weise gestoppt bzw. korrigiert werden.

Soweit das System auch die Art des medizinischen Notfalls genauer festlegen kann, können auch diese Informationen an die Rettungsleitstelle bzw. Zentrale 450 übermittelt werden. Außerdem besteht die Möglichkeit, direkt die erfassten Messdaten mit zu senden, so dass eine Fachperson eine manuelle Einschätzung der erfassten Hirnaktivität oder des sonstigen Zustandsprofils vornehmen kann, oder um beispielsweise Hirnmagnetfeld-Verläufe durch eine Recheneinheit mit mehr Ressourcen erneut ausführlich auszuwerten. Dabei könnte festgelegt sein, dass alle Daten übermittelt oder gespeichert werden, seit dem Zeitpunkt, an dem der auffällige Zustand detektiert wurde oder kurz davor; wahlweise könnte aber auch eine feste Zeitspanne vorgegeben sein, deren Zustands- und Messdaten in einem solchen Fall übermittelt werden sollen, z.B. alle erfassten Daten der letzten zehn Minuten.

Es versteht sich, dass diese Option auch genutzt werden kann, wenn kein unmittelbarer Notfall vorliegt. Beispielsweise könnte das System Messdaten, die nicht klar einem bestimmten Zustand zugeordnet werden können, aber auch von der erwarteten Hirnaktivität im Normalzustand eines Fahrers abweichen, an eine derartige Zentrale übermitteln, wo sie dann automatisch oder manuell weiter überprüft und ausgewertet werden können. Falls Zustands- und Messdaten auf diese Weise zusätzlich ausgewertet wurden, kann das Ergebnis dieser Auswertung optional auch zurück an das Fahrzeugsystem übermittelt werden, wo es dann beispielsweise zur Verbesserung zukünftiger Zustandserkennungen dienen kann.

Ebenso ist auch denkbar, dass die Messdaten aus den Magnetfeldmessungen und/oder anderen im Fahrzeug genutzten Sensoren kontinuierlich oder stichprobenartig lokal abgespeichert, an ein anderes lokales Gerät übermittelt oder über eine Kommunikationsschnittstelle an eine entfernte Einheit 450 übersendet werden, beispielsweise zur dauerhaften Speicherung in einer Speichereinheit 452. Dabei können wie bei der Notfallübermittlung von Daten optional auch bestimmte Zeitspannen festgelegt sein, über die eine Datenspeicherung stattfindet, z.B. eine lokale Speicherung, die jeweils die Daten des letzten Tages oder der letzten Fahrt umfasst. Anschließend können diese Daten an einen anderen Ort übermittelt oder überschrieben werden.

Außerdem können solche Daten auch lokal oder in entfernten Einheiten verwendet werden, um ein künstlich lernendes Modell, das zur Auswertung der gemessenen Signalverläufe verwendet wird, zu trainieren oder weiter zu verbessern. Damit kann die Auswertung auch im aktiven Fahrbetrieb kontinuierlich angepasst werden.

Als weitere Option können Informationen über die Art des Notfalls oder direkt die erfassten Messdaten der Magnetfeldsensoren oder Gyroskope lokal an eine andere Einheit 460 übermittelt werden. Auch hier kann eine geeignete Kommunikationsschnittstelle genutzt werden, wie etwa alle kurzreichweitigen Funkschnittstellen oder auch drahtgebundene Schnittstellen. Die andere Einheit 460 kann beispielsweise ein mobiles Gerät wie ein Smartphone oder ein Tablet sein, auf der die Daten auch teilweise noch weiter ausgewertet werden oder anderweitig verarbeitet werden können. Es kann sich aber auch um ein fest im Fahrzeug befindliches System handeln. Darüber hinaus ist aber auch denkbar, dass das System dazu eingerichtet ist, die Zustandsdaten und/oder Messdaten spezifisch an Auswertungsgeräte 460 zu übermitteln, die beispielsweise durch Rettungskräfte verwendet werden und an die das System automatisch oder auf Anforderung die Zustands- und/oder Messdaten übermittelt.

Die Kommunikation zu anderen Fahrzeugen 470 kann ebenfalls als Reaktion auf bestimmte erkannte Fahrerzustände genutzt werden. Falls beispielsweise eine Möglichkeit vorhanden ist, mit Fahrzeugen 470 (bzw. der in diesen Fahrzeugen vorgesehenen Steuer- und Kommunikationseinheiten 472) in einem gewissen Umkreis zu kommunizieren, kann ein erkannter Notfallzustand eines Fahrers an diese Fahrzeuge weitergemeldet werden, beispielsweise im Sinne einer Broadcast-Nachricht, die an alle erreichbaren Fahrzeuge übermittelt wird, ohne einen spezifischen Empfänger festzulegen. Die Übermittlung kann beispielsweise in Form einer Textnachricht geschehen, in der auf den Notfall hingewiesen wird und die den Passagieren in den anderen Fahrzeugen auf geeignete Weise angezeigt oder als Sprachnachricht ausgegeben werden. Damit können Fahrer in den umliegenden Fahrzeugen zum einen mit erhöhter Aufmerksamkeit auf unerwartete Fahrmanöver des erkrankten bzw. fahruntüchtigen Fahrers reagieren, können aber auch selbst möglicherweise unmittelbar erste Hilfe leisten oder andere Helfer alarmieren.

Zusätzlich oder alternativ kann eine Information über den Notfallzustand eines Fahrers aber auch an andere Fahrzeuge 470 so weitergeleitet werden, dass diese selbst automatisch reagieren können und die anderen Fahrer bzw. Passagiere nicht unbedingt involviert werden. Beispielsweise könnten Fahrzeuge, die eine solche Notfallinformation über ein Fahrzeug in ihrer Umgebung erhalten, automatisch einen größeren Abstand zu allen anderen Fahrzeugen einhalten, indem eine entsprechende autonome Abstandsregelung mit veränderten Parametern betrieben wird. Es ist dabei zwar möglich, aber nicht unbedingt nötig, dass die Benachrichtigung an die anderen Fahrzeuge weitere Informationen zu der Art des Notfalls oder der Art und Weise der erwarteten Beeinträchtigung der Fahrfunktionen umfasst.

Falls das Fahrzeug 400, in dem der Fahrerzustand überwacht wird, in Reaktion auf einen erkannten Fahrerzustand selbst bestimmte Fahrfunktionen übernimmt oder verändert, können diese Informationen teilweise oder vollständig an andere Fahrzeuge und ihre Steuerungen übermittelt werden. Die bereits vorstehend beschriebenen Reaktionen in Bezug auf die Fahrfunktionen, z.B. ein automatisches Abbremsen, ein Verringern der Geschwindigkeit, oder ein autonomes Abfahren auf eine Standspur oder Parkplatz, können entweder allgemein oder konkret durch Angabe der aktuellen Fahrzeugposition und/oder des durchgeführten Fahrmanövers an die umliegenden Fahrzeuge gemeldet werden. Dies erhöht die Sicherheit für alle beteiligten Fahrzeuge, da mit diesen Informationen - falls entsprechende Systeme in den übrigen Fahrzeugen vorhanden sind - können die anderen Fahrzeuge ebenfalls teilautonom oder autonom auf diese Fahrsituationen reagieren, so dass beispielsweise ein Auffahrunfall bei plötzlichem Abbremsen des vorderen Fahrzeugs durch einen Notfall vermieden werden kann. Als weiteres Beispiel könnte in einem Fahrzeug, das mit einem hier beschriebenen System ausgerüstet ist, die Geschwindigkeit aufgrund von erkannter Müdigkeit automatisch begrenzt werden. Wenn diese Geschwindigkeitsbegrenzung an umliegende Fahrzeuge gemeldet wird, können diese beispielsweise bei ihrer eigenen Geschwindigkeitsregelung, bei der Einhaltung eines Sicherheitsabstands zu anderen Fahrzeugen oder bei Spurwechselassistenten diese Information berücksichtigen. In anderen Fällen könnten damit andere Fahrzeuge ebenfalls generell ihre Höchstgeschwindigkeit senken, um besser auf unvorhergesehene Manöver reagieren zu können, oder sogar ebenfalls autonom von der Straße abfahren und die Gefährdungssituation abwarten.

Dabei kann die Kommunikation an andere Fahrzeuge unmittelbar als Reaktion auf einen bestimmten Fahrerzustand erfolgen und diesem zugeordnet werden. Zusätzlich oder alternativ kann aber auch eine mehrstufige Reaktion erfolgen, so dass die Übermittlung an andere Fahrzeuge nicht direkt von dem jeweiligen Fahrerzustand abhängig gemacht wird, sondern vielmehr von der Art der Systemreaktion, die auf einen beeinträchtigten Fahrerzustand hin ausgeführt wird. Falls also beispielsweise ein bewusstloser Fahrer erkannt wird, können als Reaktionen für diesen Fahrerzustand unmittelbar sowohl lokale Fahrreaktionen (Abbremsen, an den Rand fahren) als auch Benachrichtigungen anderer Fahrzeuge über diesen Zustand erfolgen. Ebenso könnte aber auch als Reaktion auf den erkannten Zustand "bewusstloser Fahrer" zunächst die lokale Fahrreaktion festgelegt werden und dann den übrigen Fahrzeugen Benachrichtigungen über die Art der Fahrreaktion übermittelt werden, z.B. Geschwindigkeits- und Positionsparameter. Es versteht sich, dass die verschiedenen Reaktionen in Bezug auf Kommunikation mit anderen Einheiten, wie etwa anderen Fahrzeugen, Rettungsdiensten, Überwachungszentralen oder anderen unabhängig davon sind, welche Kommunikationsschnittstellen verwendet werden. Die hier beschriebenen Schnittstellen und Mitteilungsarten sind nur beispielhaft zu verstehen und können durch beliebige geeignete Datenformate und Schnittstellen, die dem System zur Verfügung stehen, ersetzt oder erweitert werden.

Es ist möglich, jedem spezifischen mentalen bzw. gesundheitlichen Zustand des Fahrers auch eine konkrete Reaktion oder eine Gruppe von Reaktionen zuzuweisen. Eine solche Zuordnung kann beispielsweise einfach in einer Zuordnungstabelle festgelegt und abgespeichert sein.

Ebenso ist aber auch möglich, dass die bekannten Fahrerzustände in vorgegebene Zustandsgruppen sortiert sind und jeder Gruppe eine oder mehrere fest vorgegebene Reaktionen zugeordnet sind. Beispielsweise könnten die Fahrerzustände in die Gruppen "erhöhte Aufmerksamkeit nötig", "medizinischer Notfall", "medizinischer Notfall mit Bewusstlosigkeit", "unklarer Zustand" eingeteilt werden. Die daraufhin eingeleiteten Reaktionen könnten dann jeder dieser Zustandsgruppen zugeordnet werden, und falls einer der Zustände in einer Gruppe erkannt wird, können die zu dieser Zustandsgruppe zugehörigen Reaktionen im Fahrzeug durchgeführt werden. Dabei handelt es sich bei diesen Einteilungen natürlich nur um Beispiele, so dass die Gruppeneinteilung auch völlig anders ausgestaltet werden können. Es könnten auch Zustände mehreren Zustandsgruppen und den zugehörigen Reaktionen zugeordnet sein.

Es können darüber hinaus optional Reaktionen festgelegt sein, die immer gemeinsam ausgelöst werden, wie etwa das plötzliche autonome Abbremsen durch einen Bremsassistenten im Fahrzeug, das immer mit einer Information der umliegenden Fahrzeuge durch Vehicle-to-Vehicle-Kommunikation sowie einem hörbaren oder sichtbaren Warnsignal kombiniert wird, wie blinkenden Warnleuchten. Beliebige andere Kombinationen von Reaktionen sind möglich.

Optional kann bei allen beliebigen Reaktionen auch vorgesehen sein, dass dem Fahrer die automatische Aktivierung bestimmter Reaktionen (Notruf, Fahrfunktionen) zunächst durch Anzeige oder Sprachausgabe angezeigt wird und dass der Nutzer dann durch entsprechende Eingabemittel, z.B. eine Spracheingabe, noch in die Reaktionen eingreifen kann oder das Auslösen der geplanten Reaktionen vollständig stoppen kann. Auf diese Weise kann ein Fehlalarm, der möglicherweise kritische Reaktionen auslöst, vermieden werden.

Die vorstehenden Beispiele wurden vor allem in Bezug auf Kraftfahrzeuge wie Autos beschrieben. Allerdings können die beschriebenen Ausführungsformen ebenso auf Führer anderer Fahrzeuge (Landfahrzeuge, Wasserfahrzeuge und Luftfahrzeuge aller Art), wie etwa Piloten, Zugführer, Kapitäne, Busfahrer, oder Fernlastfahrer oder allgemein auf Bediener anderer sicherheitskritischer Einrichtungen übertragen werden. Die Reaktionen wie Wecksignale, Alarmsignale oder Fernalarmierungen von Rettungskräften können auch in diesen Fällen wie beschrieben angewendet werden. Der Fachmann wird in der Lage sein, die jeweiligen verwendeten Einheiten auf solche anderen Anwendungsbereiche zu übertragen. Bei Piloten eines Luftfahrzeugs kann beispielsweise im Notfall als Reaktion festgelegt sein, dass ein Co-Pilot durch akustische oder optische Hinweise zur Übernahme aller Funktionen angewiesen und berechtigt wird. Auch die Verbindung des Systems mit Infrastrukturelementen, ähnlich der Vehicle-to-Infrastructure-Funktion im Straßenverkehr, kann entsprechend auf andere Bereiche übertragen werden, z.B. bei einem Zugführer durch zusätzliche Meldung von Notfällen an ein Stellwerk, das für die befahrene Zugstrecke zuständig ist.

Es versteht sich, dass alle vorstehend in einzelnen Beispielen beschriebenen Elemente auch beliebig kombinierbar sind. So sind alle beschriebenen Arten der Fahrerzustandserkennung, einzeln oder in Kombination, mit allen der genannten Reaktionen kombinierbar. Auch Änderungen an den beispielhaft beschriebenen Sensorelementen und den zugehörigen Aufbauten (z.B. an Sensorelektronik, Sensoraufbau, Halterungen), sind möglich, ohne von dem grundlegenden Prinzip der zustandsabhängigen Reaktion im Fahrzeug abzuweichen.

**Patentansprüche**

1. Verfahren zur Zustandsüberwachung eines Insassen in einem Fahrzeug (400), umfassend:

Erfassen (300), über eine oder mehrere Sensoreinheiten (101, 102, 401) im Kopfbereich des Insassen, von Sensordaten, wobei die Sensordaten mindestens zwei Feldstärken von magnetischen Feldern an jeweils räumlich unterschiedlichen Stellen im Kopfbereich des Insassen umfassen,

Bildung mindestes eines Gradienten aus den mindestens zwei erfassten Feldstärken, so dass ein magnetisches Hintergrundfeld im Wesentlichen eliminiert wird;

Prüfen (330), ob der gebildete Gradient auf einen unerwünschten oder unbekannten Zustand des Insassen hinweist, der die Fahrtüchtigkeit des Insassen einschränkt;

und falls dies der Fall ist, Auslösen einer zustandsabhängigen Reaktion (350, 360) in dem Fahrzeug, wobei

die zustandsabhängige Reaktion (350, 360) mindestens eines der folgenden umfasst: Aktivieren einer autonomen oder teilautonomen Fahrfunktion (430) des Fahrzeugs; Deaktivieren einer autonomen oder teilautonomen Fahrfunktion des Fahrzeugs; Verändern von Betriebsparametern einer autonomen oder teilautonomen Fahrfunktion des Fahrzeugs.

2. Verfahren nach Anspruch 1, wobei die zustandsabhängige Reaktion (350, 360) in dem Fahrzeug (400) das Ausgeben eines optischen oder akustischen Signals (420, 422) umfasst.

3. Verfahren nach Anspruch 2, wobei das optische oder akustische Signal einen Hinweis auf die Art der auszulösenden zustandsabhängigen Reaktion und/oder einen Hinweis auf den erkannten Zustand des Insassen umfasst.

4. Verfahren nach einem der vorhergehenden Ansprüche, wobei die zustandsabhängige Reaktion nicht ausgelöst wird, falls ein Befehl zum Abbrechen der Reaktion erfasst wird.

5. Verfahren nach einem der vorhergehenden Ansprüche, wobei die autonome oder teilautonome Fahrfunktion (430) des Fahrzeugs mindestens eines der folgenden umfasst: eine Abstandsregelung zum Regeln eines Abstands des Fahrzeugs von anderen Objekten; eine Regelung der Fahrgeschwindigkeit des Fahrzeugs; eine vollautonome Steuerung des Fahrzeugs.

6. Verfahren nach einem der vorhergehenden Ansprüche, wobei die zustandsabhängige Reaktion ein Übermitteln einer Nachricht über eine Kommunikationsschnittstelle (440) umfasst.

7. Verfahren nach Anspruch 6, wobei die Nachricht zumindest einen Teil der erfassten Sensordaten und/oder Informationen über die erfassten Sensordaten umfasst.

8. Verfahren nach Anspruch 6 oder 7, wobei die Nachricht an weitere Fahrzeuge (470) übermittelt wird, die sich in der Umgebung des Fahrzeugs (400) befinden.

9. Verfahren nach einem der Ansprüche 6 bis 8, wobei die Nachricht Informationen über das Aktivieren, Deaktivieren oder Verändern einer Fahrfunktion (430) umfasst.

10. Verfahren nach einem der Ansprüche 6 bis 9, wobei die Nachricht eine Notrufnachricht an eine Notrufzentrale (450) umfasst.

11. Verfahren nach einem der vorhergehenden Ansprüche, wobei der unerwünschte Zustand des Insassen mindestens eines der folgenden umfasst: Müdigkeit, Einschlafen, erhöhtes Stresslevel, niedrige Konzentration, eine neuronale Erkrankung, einen epileptischen Anfall, einen Schlaganfall.

12. Verfahren nach einem der vorhergehenden Ansprüche, wobei mehrere Gruppen von möglichen Zuständen eines Insassen vorgegeben sind, und wobei jeder Gruppe eine oder mehrere Reaktionen zugeordnet sind.

13. Verfahren nach einem der vorhergehenden Ansprüche, wobei die mindestens eine Sensoreinheit (101, 102, 401) einen spinresonanzbasierten Magnetfeldsensor zum Erfassen von durch Hirnströmen induzierten Hirn-Magnetfeldern und/oder ein Gyroskop zum Erfassen von Kopfbewegungen umfasst.

14. Recheneinheit (410), die dazu eingerichtet ist, alle Verfahrensschritte eines Verfahrens nach einem der vorstehenden Ansprüche durchzuführen.

15. Computerprogramm, das eine Recheneinheit dazu veranlasst, alle Verfahrensschritte eines Verfahrens nach einem der Ansprüche 1 bis 13 durchzuführen, wenn es auf der Recheneinheit ausgeführt wird.

16. Maschinenlesbares Speichermedium mit einem darauf gespeicherten Computerprogramm nach Anspruch 15.

**Claims**

1. Method for monitoring the condition of an occupant in a vehicle (400), comprising:

capturing (300) sensor data via one or more sensor units (101, 102, 401) in the head region of the occupant, wherein the sensor data comprise at least two field strengths of magnetic fields at respective spatially different locations in the head region of the occupant,

forming at least one gradient from the at least two captured field strengths, such that a magnetic background field is substantially eliminated;

checking (330) whether the gradient formed is indicative of an undesirable or unknown condition of the occupant that restricts the occupant's driving ability;

and, if so, triggering a condition-dependent response (350, 360) in the vehicle, wherein

the condition-dependent response (350, 360) comprises at least one of the following: activating an autonomous or partially autonomous driving function (430) of the vehicle; deactivating an autonomous or partially autonomous driving function of the vehicle; changing operating parameters of an autonomous or partially autonomous driving function of the vehicle.

2. Method according to Claim 1, wherein the condition-dependent response (350, 360) in the vehicle (400) comprises outputting an optical or acoustic signal (420, 422).

3. Method according to Claim 2, wherein the optical or acoustic signal comprises an indication of the type of condition-dependent response to be triggered and/or an indication of the detected condition of the occupant.

4. Method according to one of the preceding claims, wherein the condition-dependent response is not triggered if a command to abort the response is captured.

5. Method according to one of the preceding claims, wherein the autonomous or partially autonomous driving function (430) of the vehicle comprises at least one of the following: distance regulation for regulating a distance of the vehicle from other objects; regulation of the driving speed of the vehicle; fully autonomous control of the vehicle.

6. Method according to one of the preceding claims, wherein the condition-dependent response comprises transmitting a message via a communication interface (440).

7. Method according to Claim 6, wherein the message comprises at least a portion of the captured sensor data and/or information about the captured sensor data.

8. Method according to Claim 6 or 7, wherein the message is transmitted to further vehicles (470) located in the surroundings of the vehicle (400).

9. Method according to one of Claims 6 to 8, wherein the message comprises information about the activation, deactivation or modification of a driving function (430).

10. Method according to one of Claims 6 to 9, wherein the message comprises an emergency call message to an emergency call centre (450).

11. Method according to one of the preceding claims, wherein the undesirable condition of the occupant comprises at least one of the following: fatigue, falling asleep, increased stress level, low concentration, a neuronal disease, an epileptic fit, a stroke.

12. Method according to one of the preceding claims, wherein a plurality of groups of possible conditions of an occupant are predefined, and wherein one or more responses are assigned to each group.

13. Method according to one of the preceding claims, wherein the at least one sensor unit (101, 102, 401) comprises a spin-resonance-based magnetic field sensor for capturing brain magnetic fields induced by brain waves and/or a gyroscope for capturing head movements.

14. Computing unit (410) which is configured to carry out all of the method steps of a method according to one of the preceding claims.

15. Computer program that prompts a computing unit to carry out all of the method steps of a method according to one of Claims 1 to 13 when it is executed on the computing unit.

16. Machine-readable storage medium with a computer program according to Claim 15 stored thereon.

**Revendications**

1. Procédé de surveillance de l'état d'un occupant dans un véhicule (400), comprenant :

l'acquisition (300) de données de capteur, par l'intermédiaire d'une ou de plusieurs unités de détection (101, 102, 401) dans la région de la tête de l'occupant, les données de capteur comprenant au moins deux intensités de champ de champs magnétiques à des emplacements spatialement différents respectifs dans la région de la tête de l'occupant,

le calcul d'au moins un gradient à partir des au moins deux intensités de champ acquises, de sorte qu'un champ magnétique d'arrière-plan soit sensiblement éliminé ;

la vérification (330) du fait de savoir si le gradient calculé indique un état indésirable ou inconnu de l'occupant, lequel état limite le confort de conduite de l'occupant ;

et si tel est le cas, le déclenchement d'une réponse dépendant de l'état (350, 360) dans le véhicule,

la réaction dépendant de l'état (350, 360) comprenant au moins l'un des éléments suivants : activation d'une fonction de conduite autonome ou semi-autonome (430) du véhicule ; désactivation d'une fonction de conduite autonome ou semi-autonome du véhicule ; modification de paramètres de fonctionnement d'une fonction de conduite autonome ou semi-autonome du véhicule.

2. Procédé selon la revendication 1, dans lequel la réponse dépendant de l'état (350, 360) dans le véhicule (400) comprend la fourniture en sortie d'un signal optique ou acoustique (420, 422).

3. Procédé selon la revendication 2, dans lequel le signal optique ou acoustique comprend une indication du type de réponse dépendant de l'état à déclencher et une indication de l'état détecté de l'occupant.

4. Procédé selon l'une des revendications précédentes, dans lequel la réponse dépendant de l'état n'est pas déclenchée si une instruction d'abandon de la réponse est acquise.

5. Procédé selon l'une des revendications précédentes, dans lequel la fonction de conduite autonome ou partiellement autonome (430) du véhicule comprend au moins l'un des éléments suivants : une régulation de distance pour réguler une distance du véhicule par rapport à d'autres objets ; une régulation de la vitesse de conduite du véhicule ; une commande entièrement autonome du véhicule.

6. Procédé selon l'une des revendications précédentes, dans lequel la réaction dépendant de l'état comprend la transmission d'un message par l'intermédiaire d'une interface de communication (440).

7. Procédé selon la revendication 6, dans lequel le message comprend au moins une partie des données de capteur acquises et/ou des informations concernant les données de capteur acquises.

8. Procédé selon la revendication 6 ou 7, dans lequel le message est transmis à d'autres véhicules (470) qui se trouvent dans l'environnement du véhicule (400).

9. Procédé selon l'une des revendications 6 à 8, dans lequel le message comprend des informations concernant l'activation, la désactivation ou la modification d'une fonction de conduite (430).

10. Procédé selon l'une des revendications 6 à 9, dans lequel le message comprend un message d'appel d'urgence destiné à un centre d'appels d'urgence (450).

11. Procédé selon l'une des revendications précédentes, dans lequel l'état indésirable de l'occupant comprend au moins l'un des éléments suivants : de la fatigue, un endormissement, une augmentation du niveau de stress, une faible concentration, une maladie neuronale, une crise d'épilepsie, un accident vasculaire cérébral.

12. Procédé selon l'une des revendications précédentes, dans lequel plusieurs groupes d'états possibles d'un occupant sont prédéfinis et dans lequel une ou plusieurs réactions sont associées à chaque groupe.

13. Procédé selon l'une des revendications précédentes, dans lequel ladite au moins une unité de détection (101, 102, 401) comprend un capteur de champ magnétique basé sur la résonance de spin pour détecter des champs magnétiques cérébraux induits par des courants cérébraux et/ou un gyroscope pour détecter des mouvements de tête.

14. Unité de calcul (410) qui est conçue pour exécuter toutes les étapes de procédé d'un procédé selon l'une des revendications précédentes.

15. Programme informatique qui amène une unité de calcul à mettre en œuvre toutes les étapes de procédé d'un procédé selon l'une des revendications 1 à 13, lorsqu'il est exécuté sur l'unité de calcul.

16. Support de stockage lisible par machine, sur lequel est enregistré un programme informatique selon la revendication 15.

FIG. 1

**FIG. 2**

# FIG. 3

# FIG. 4

EP 4 479 959 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2017305349 A
- US 20200057115 A
- US 20150219732 A
- DE 102018202238 A1
- DE 102019219061 A1